# EUROPÄISCHE PATENTSCHRIFT

(11) **EP 1 654 163 B1**
(45) Veröffentlichungstag und Bekanntmachung des Hinweises auf die Patenterteilung: **07.03.2007**
(21) Anmeldenummer: 04803590.1
(22) Anmeldetag: 07.12.2004
(51) Int. Cl.: B65D 1/24, B01L 9/06, G01N 35/00, B65B 21/14

(54) **TRANSPORTBEHÄLTER**
TRANSPORT CONTAINER
CAISSON DE TRANSPORT

(30) Priorität: 28.01.2004 DE 102004004324
(43) Veröffentlichungstag der Anmeldung: 10.05.2006
(73) Patentinhaber: Bartec GmbH, 97980 Bad Mergentheim (DE)
(72) Erfinder: BÖHM, Alfred, 94234 Viechtach (DE); GRÜNERT, Rudolf, 94315 Straubing (DE); VÖLKL, Franz, 9423 Viechtach (DE)
(74) Vertreter: Heim, Hans-Karl
(86) Internationale Anmeldenummer: PCT/EP2004/013906
(87) Internationale Veröffentlichungsnummer: WO 2005/073097

(56) Entgegenhaltungen:
- WO-A-92/18391
- CH-A- 537 842
- DE-A1- 2 254 368
- FR-A- 2 180 438
- US-A- 3 841 519
- US-A1- 2003 143 749

## Beschreibung

Die Erfindung betrifft einen Transportbehälter gemäß Oberbegriff des Anspruchs 1 und ein Verfahren zum Entleeren eines Transportbehälters gemäß Oberbegriff des Anspruchs 18.

Transportbehälter und Verfahren dieser Art sind insbesondere bei Milchprobenflaschen, welche zur Qualitätskontrolle bei der Milchübernahme eingesetzt werden, bekannt. Insbesondere beim Lebensmittel Milch, die einer permanenten strengen Qualitätskontrolle unterliegt, werden Probenflaschen eingesetzt, in die bei der Annahme der Milch in Milchsammelwagen, z.B. 25 ml Milch, in die Probenflasche abgezweigt wird. Diese Proben werden anschließend im Labor analysiert. Teilweise werden sie auch im Rahmen einer Milchleistungsprüfung untersucht, um Aussagen über die Milchleistung von Kühen treffen zu können.

Bereits in Deutschland werden täglich zig-tausend solcher Proben genommen, wobei durch die zunehmende Zentralisierung der Labors die Milchprobenflaschen und deren Transportbehälter für eine weitgehend automatische Handhabung geeignet sein sollten. Die manuelle Betätigung darf hierbei jedoch nicht ausgeschlossen werden.

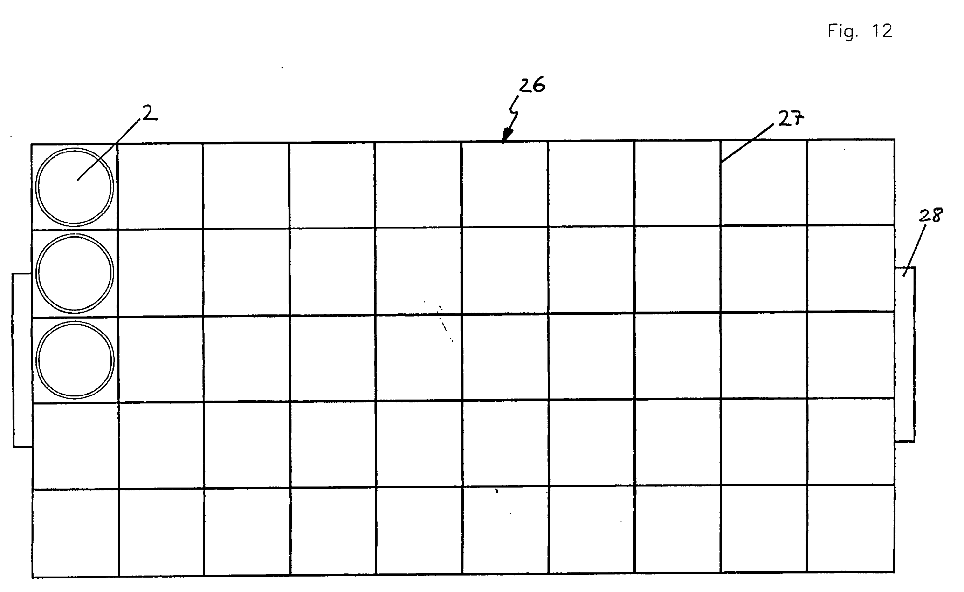

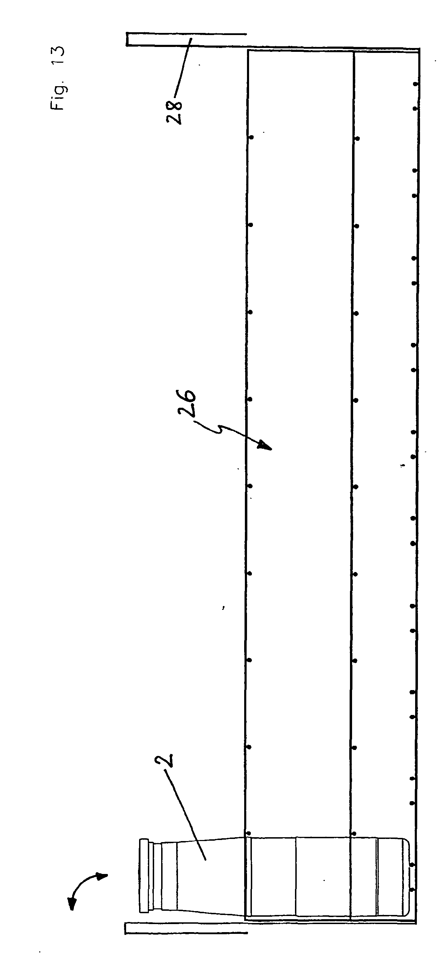

Es sind daher Transportbehälter bekannt, wie sie in den Figuren 12 bis 15 schematisch dargestellt sind. Diese Körbe 26 können Traggriffe 28 aufweisen und sind üblicherweise mit einem Lochblech oder einer Gitterstabeinteilung 27 ausgestattet. Üblicherweise werden hierbei manuell die entsprechenden Probenflaschen 2 in die einzelnen Anordnungsplätze eingesetzt und bei Bedarf daraus auch wieder manuell entfernt.

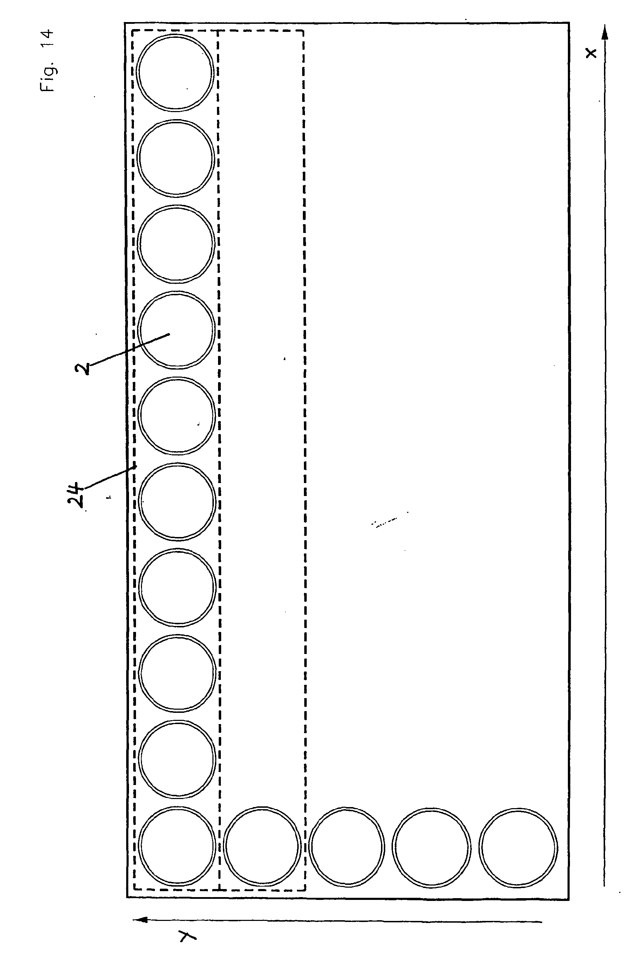

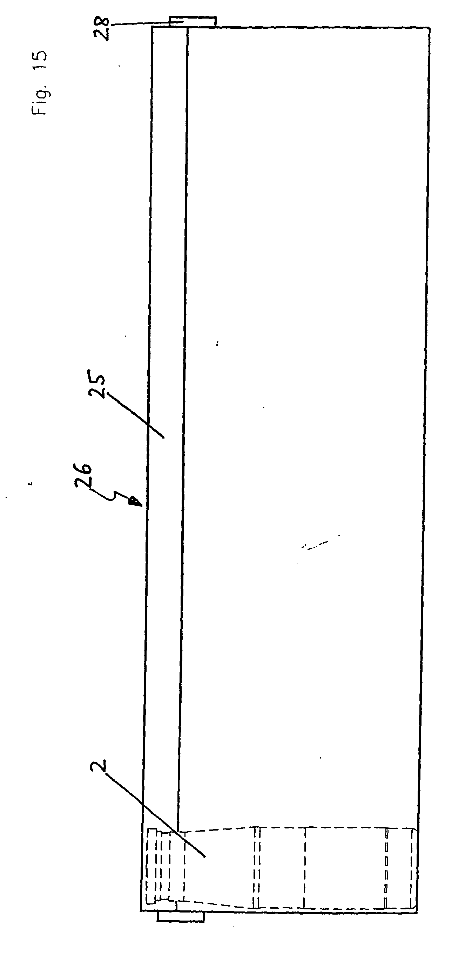

Eine weitere Form dieser Transportbehältnisse ist schematisch in den Figuren 14, 15 dargestellt. Dort sind einzelne Linearmagazine 24, in denen eine Anzahl von Probenflaschen 2 hintereinander vorgesehen sind, in einen Korb oder ein Behältnis 26 eingesetzt, wobei mehrere dieser Linearmagazine 24 darin aufgenommen werden können. Das Behältnis 26 kann, wie in Figur 15 dargestellt, auch mit einem Deckel 25 versehen sein, der aufklappbar und auch versiegelbar oder mit einem Klappverschluss angeordnet werden kann.

Ein Transportbehälter gemäß Oberbegriff des Anspruch 1 wird durch CH 537842 offenbart.

Weiterhin sind auch Rundmagazine bekannt, z.B. DE 34 15 873 A1, die zwar eine weitgehend automatische Handhabung der Probenflaschen erlauben. Diese Rundmagazine benötigen jedoch relativ großen Platz und sind auch vergleichsweise schwer und teuer.

Aus der DE 1 099 999 C ist eine Transportkiste bzw. ein Flaschenschrank bekannt, bei dem ein Seitenteil abgeklappt werden kann, um so die Flaschen besser handhaben zu können. Ein ähnliches Prinzip ist in der DE 236 839 C beschrieben. Hierbei handelt es sich um eine Gelenkbandkiste, bei der ebenfalls ein Seitenteil abklappbar ist. Eine automatisierte Handhabung von Flaschen ist bei diesen Transportkisten jedoch nicht möglich.

Aus CH 690 648 A5 ist ein Einsatzteil bekannt, das in einen Behälter aus Kunststoff eingesetzt werden kann, um Objekte darin leichter zu positionieren. Eine eventuelle automatisierte Handhabung der Objekte wird durch das Einsatzteil nicht erleichtert. Im Gegenteil, zum Entfernen der Objekte wäre ein komplexes System zum einzelnen Greifen notwendig.

Die DE 44 36 473 C2 offenbart eine Bereitstellungs- und -entnahmevorrichtung für Pipettenspitzen. Hierbei werden die Pipettenspitzen in eine Lochplatte gesteckt und in Lagen als Magazin platzsparend übereinander angeordnet. Mit dieser Vorrichtung soll erreicht werden, dass Pipettenspitzen bequem gehandhabt werden können und damit ein großes Magazin für das Lagern dieser Spitzen bestückt werden kann. Diese Druckschrift zielt vor allem auf den Laborbetrieb ab. Hierbei wird weniger auf eine Handhabung durch Maschinen, als eine einfache Handhabung und Bevorratung für den menschlichen Benutzer geachtet.

Ferner ist aus DE 44 43 775 C2 eine Verpackungseinheit bekannt, die selbsttätig die in ihr gelagerten Waren nach vorne schiebt. Dies dient dem Zweck, dass bei einer in einem Ladenregal vorhanden Verpackungseinheit, die Waren immer nach vorne geschoben werden, so dass diese für den Kunden leichter entnehmbar sind. Hierbei besteht keine Möglichkeit dies gesteuert, oder nur zu bestimmten Zeiten auszuführen.

Aus DE 78 29 969 Ul ist ein tragbarer Behälter für Mappen, Akten oder dergleichen bekannt. Der Behälter, der aus zwei Teilen besteht, nämlich einer Hülse und dem eigentlichen Behälter, wird ineinander geschoben, so dass die transportierten Akten geschützt sind. Eine automatisierte Handhabung von Flaschen ist mit diesem Behälter ebenfalls nicht möglich.

Ein Verfahren zum Entleeren eines Transportbehälters gemäß Oberbegriff des Anspruchs 18 wird durch FR 2 180 438 offenbart.

Unter Berücksichtigung dieses Standes der Technik ist es **Aufgabe** der Erfindung, einen Transportbehälter für Probenflaschen zu schaffen, der für eine Automatisierung geeignet ist, aber auch manuell bedienbar ist, wobei Raumersparnis, preiswerte Konzeption, gute Reinigbarkeit und Modulcharakter hinzutreten.

Diese Aufgabe wird erfindungsgemäß bei einem Transportbehälter durch die Merkmale des Anspruchs 1 und bei einem Verfahren mittels der Merkmale des Anspruchs 18 gelöst.

Ein Kerngedanke der Erfindung ist hierbei, einen Transportbehälter mit ebener Abstellfläche für die Probenflaschen zu schaffen, ohne Mittel zur formschlüssigen Einzelplatzierung der Probenflaschen vorzusehen und beim Entleerungsvorgang mindestens eine Seitenwand entfernbar anzuordnen, so dass die Probenflaschen einzeln, reihenweise oder blockweise aus dem Transportbehälter ausgeschoben werden können. Es kann bereits allein durch eine Schrägstellung des Transportbehälters und dann schwerkraftmäßig die Abgabe und Überführung der Probenflaschen auf eine direkt anliegende Fördereinheit realisiert werden.
Trotzdem ist eine manuelle Handhabung noch möglich.

Anstelle von Probeflaschen können hierbei auch andere Behältnisse, z.B. runde Dosen, quaderartige Behältnisse oder dergleichen, soweit diese gleitfähig sind, verwendet werden.

Verfahrensmäßig wird vorgesehen, dass mindestens eine Seitenwand nach außen oder oben geöffnet werden kann und die flaschenartigen Behälter oder Probenflaschen zur weiteren Förderung und Analyseuntersuchung abgegeben werden können.

Hierbei wird bei der automatischen Handhabung, insbesondere von einer berührenden, benachbarten Platzierung der Probenflaschen innerhalb des Transportbehälters ausgegangen, so dass Flaschenführungselemente bzw. Führungsstege entfallen können.

Diese Transportbehälter eignen sich auch für Probenflaschen, flaschenartige Behältnisse oder Behältnisse für andere Medien als Lebensmittel, z.B. chemische oder pharmazeutische Erzeugnisse, Wasserproben etc., welche automatisch einer weiteren Verarbeitung oder Untersuchung zugeführt werden sollen.

Die Transportbehälter sind auch als Getränke- oder Bierkästen geeignet.

Die normalerweise aus einem Kunststoff bestehenden Transportbehälter sind für den Zweck der Handhabung mit Probenflaschen vorteilhafterweise mit einem Deckel ausgestattet. In diesem Deckel werden zur verbesserten Handhabung Öffnungen im Sinne von Pipettieröffnungen vorgesehen, die mit den kopfseitigen Verschlüssen der Probenflaschen ausgerichtet sind. Auf diese Weise lässt sich die Übernahme von Proben in die Probenflaschen, z.B. durch das Einsetzen von Injektionskanülen, durch diese Öffnungen und das Durchstoßen der Stopfen bzw. Folienabschlüsse der Probenflaschen ermöglichen. Es kann somit auch eine Kontamination zwischen einzelnen Proben ausgeschlossen werden.

Die zu öffnende Seitenwand wird konstruktiv zweckmäßigerweise so ausgelegt, dass sie über seitliche Führungsnuten in den begrenzenden Eckbereichen nach oben verschoben werden kann und anschließend eine weitgehend formschlüssige Aufnahme auf dem oder im Deckel findet.
Auch eine einfache Scharnieranlenkung in der oberen Längsachse der zu öffnenden Seitenwand ist möglich. Hierbei kann eine Verbindung mit dem Deckel durchgeführt werden, so dass Seitenwand und Deckel aufgeklappt und verschwenkbar sind und auch ein Befüllen des Transportbehälters von Hand oder automatisch von oben ermöglicht wird.

Im Hinblick auf weitere Gewichtsersparnis werden die Seitenwände nicht vollflächig mit Material gestaltet, sondern mit weitgehend horizontal verlaufenden Wandstegen und Zwischenräumen dazwischen ausgestattet. Speziell im unteren Bereich der Probenflaschen ist ein derartiger Freiraum vorzusehen, über den von außen oder unten Schiebemittel zumindest mit linienartiger oder flächenartiger Berührung mit den Probenflaschen eingeführt und die Probenflaschen durch die Öffnung der entfernten Seitenwand ausgeschoben werden können.

In einfachster Weise genügt hierzu eine leichte Schrägstellung des Transportbehälters, so dass die Probenflaschen gleitend über die geöffnete Seite, z.B. auf ein Förderband oder einen Schneckenförderer zum Weitertransport abgegeben werden können.

Zur Verbesserung der Führung innerhalb des Transportbehälterns können bodenseitig relativ niedrige Stege in Ausschubrichtung vorgesehen sein, womit bessere Führungseigenschaften und ein Ausschreiben auch reihenweise in der Tiefe, nicht nur parallel zu der entfernbaren Seitenwand, möglich ist. Dasselbe Ergebnis kann durch deckenseitige Stege erreicht werden. Zusätzlich verhindert die deckseitige Anbringung der Stege, das Umfallen der Probenflaschen während des Transports. Es ist ebenso möglich, sowohl deckenseitige als auch bodenseitige Stege vorzusehen, um eine größere Führungssicherheit beim Ausschieben zu erreichen.

Die entfernbare Seitenwand könnte auch über ein vertikal im Eckbereich vorgesehenes Scharnier, oder mittig im Sinne einer Tor-Öffnung aufschwenkbar sein.
Auch eine horizontale Verschwenkung des Deckels mit Anlenkung an einer Ecke ist denkbar.

Das Entleeren der Probenflaschen erfolgt vorteilhafterweise über ein im unteren Bereich der Probenflaschen angreifenden und durch eine freie Fläche zwischen den Seitenstegen eingeführtes Schiebemittel. Dieses kann eine Schiebefläche, im Wesentlichen parallel zur geöffneten Seitenwand, darstellen oder auch nur eine Berührungslinie mit den Probenflaschen bilden. Auch das Einführen einer Schiebeinrichtung als dünne Platte oder Stab senkrecht zur Ausschubrichtung zwischen die Reihe der Probenflaschen mit anschließender Bewegungsrichtung in Ausschubrichtung ist möglich.

Auch ein blockweises Ausschieben aller in einem Transportbehälter befindlichen Probenflaschen ist realisierbar, wobei auch eine getaktete Abgabe denkbar ist.

Auch lässt es sich realisieren, die Probenbehälter in einer geordneten Linie abzugeben, wobei das Prinzip last in - first out umgesetzt werden kann. Die Probenbehälter werden dabei z.B. getaktet aus dem Transportbehälter in eine Transportschnecke oder auf ein Lamellenband abgegeben.

Üblicherweise wird hierbei ein gleiches Niveau zwischen der externen Transportebene und dem Bodeniveau, auf welchem die Probenbehälter im Transportbehälter vorhanden sind, angestrebt.

Um Manipulation auszuschließen, ist es auch möglich, eine Versiegelung des Transportbehälters, insbesondere zwischen Deckel und zu öffnender Seitenwand vorzusehen. Dies kann in Art eines Klebesiegels, Drahtsiegels oder starren Kunststoffsiegels realisiert werden.

Obwohl die Transportbehälter auf eine unterschiedliche Anzahl von Probenflaschen ausgelegt sein können, ist angestrebt, den Transportbehälter modulartig zu konstruieren.

In diesem Sinne ist es möglich, die kleinste Einheit eines Transportbehälters mit anderen Einheiten horizontal und/oder vertiakl zu kombinieren, wobei Selbstrastmechanismen, insbesondere im Eck- oder Bodenbereich, und einsetzbare Verbindungselemente ein Stapeln und Kaskadieren mehrerer Transportbehälter zu einer größeren Transport-Einheit gestatten. Auch dies dient der Handhabungsvereinfachung.

Der Deckel ist geeigneterweise so ausgelegt, dass er eine Selbstarretierung mindestens mit einer Seitenwand aufweist.

Des Weiteren ist bei einer Aufklappbarkeit des Deckels über seine Längsachse der mit der Seitenwand in Eingriff stehende Scharnierzapfen mit einem Flachbereich versehen, so dass in entsprechender Stellung, z.B. nach einem Verschwenken von 180° , der Deckel auch vollständig entfernt werden kann.

Ebenfalls sind Kennzeichnungsfelder zur Anbringung von Code-Kennzeichnung oder TAGs vorhanden, wodurch die automatische Identifizierung möglich ist.

Bodenseitig können im Transportbehälter Öffnungen kreisförmiger Art mit Zuordnung zu einzelnen Probenflaschen, oder in Art eines Langloches oder U-förmigen Langloches vorhanden sein. Diese bodenseitigen Öffnungen erlauben auch ein vertikales Entleeren des Transportbehälters oder Befüllen über von unten eingeführte Stößel. Andererseits ist auch ein automatisches Ausschieben der Probenflaschen mit von unten durch den Boden angreifenden Schiebemitteln möglich.

Sofern ein blockweises Ausschieben durchgeführt wird, z.B. bei einem Transportbehälter mit 15 oder 30 Probenflaschen, ist auch die Anbringung eines Einlegegitters zur formschlüssigen Führung der Probenflaschen möglich. Dadurch wird ein eventuelles Vertauschen der Flaschenposition vermieden.

Die Erfindung wird nachfolgend anhand schematischer Zeichnungen noch näher erläutert. Es zeigen:
- Fig. 1: eine perspektivische Ansicht eines Transportbehälters mit geschlossener Seitenwand und geschlossenem Deckel;
- Fig. 2: einen schematischen Vertikalschnitt durch einen Teil eines derartigen Transportbehälters;
- Fig. 3: eine schematische Draufsicht auf einen Teil eines Transportbehälters ohne Deckel mit Hinweis auf die Schiebeeinrichtung;
- Fig. 4: eine perspektivische schematische Darstellung eines Transportbehälters mit zwei unterschiedlichen Schiebeeinrichtungen;
- Fig. 5: eine perspektivische Ansicht eines größeren Transportbehälters als in Figur 1 mit Blick auf die Öffnung der in den Deckelbereich nach oben verschobenen Seitenwand;
- Fig. 6: eine perspektivische schematische Ansicht des Transportbehälters nach Figur 5 mit 30 Probenflaschen im geöffneten Zustand, bei dem der Deckel und die Seitenwand noch oben aufgeschwenkt sind;
- Fig. 7: eine vertikale schematische Teilansicht eines Transportbehälters zur Verdeutlichung der Handhabung mit einer automatisch einführbaren Injektionsnadel und einer Versiegelung;
- Fig. 8: eine schematische Draufsicht auf einen Teil eines Transportbehälters mit angedeuteten Öffnungen im Boden in zwei ersten Ausführungsformen und
- Fig. 9: in vergleichbarer Weise wie Figur 8 mit zwei weiteren Ausführungsformen;
- Fig. 10: eine schematische Draufsicht auf einen Transportbehälter ohne Deckel zur reihenweisen Ausschiebung der Probenflaschen auf einen z.B. Schneckenförderer, und
- Fig. 11: eine schematische Darstellung in Art eines vertikalen Schnittes eines Transportbehälters zur Verdeutlichung des Übergabeniveaus der Probenflaschen.
- Fig. 12: zeigt die schematische Draufsicht auf einen bekannten Transportkorb und
- Fig. 13: einen Vertikalschnitt durch einen derartigen Transportkorb nach Figur 12 gemäß Stand der Technik;
- Fig. 14: eine Draufsicht in schematischer Form auf einen weiteren bekannten Transportkorb mit angedeutetem Linearmagazin sowie
- Fig. 15: eine Seitenansicht auf einen Transportkorb nach Figur 14 mit geschlossenem Deckel.

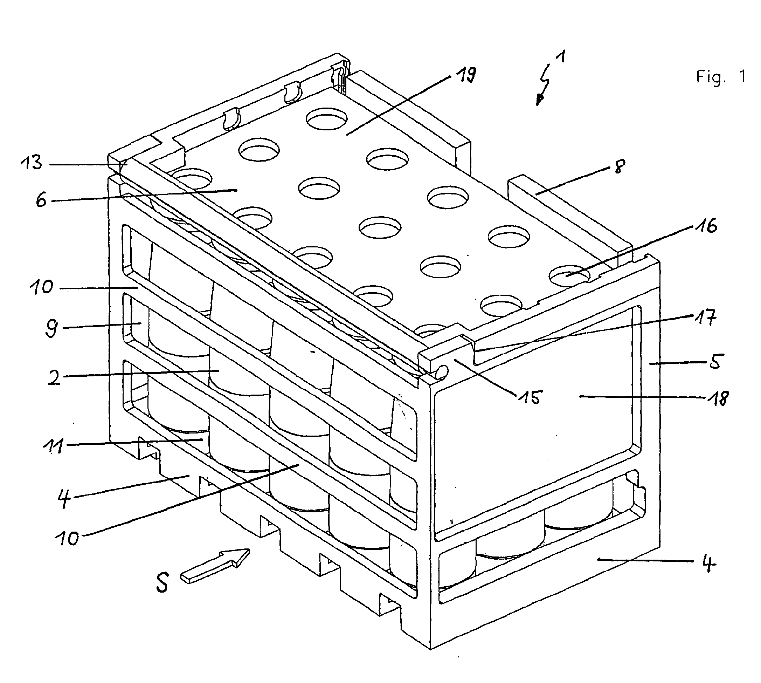

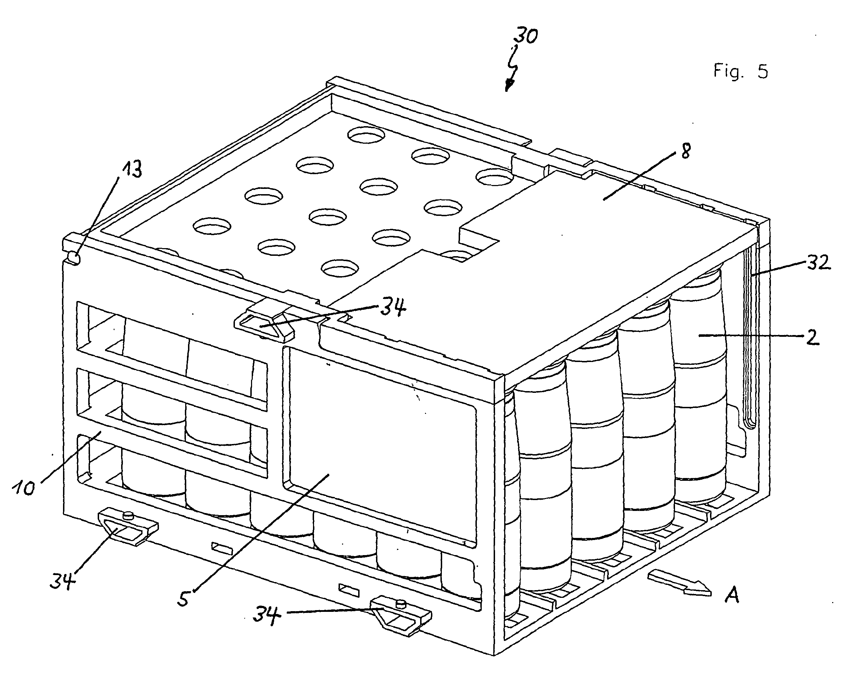

Ein erfindungsgemäßer Transportbehälter 1 ist in der Fig. 1 in perspektivischer Ansicht dargestellt. Der aus einem strapazierfähigen, leichten Material, insbesondere Kunststoff, hergestellte Transportbehälter 1 weist im Bodenbereich einen Boden 4 und in den Seitenbereichen vier Seitenwände 5, 9 und 8 auf. Die Seitenwand 9 ist hierbei aus Gewichtsersparnisgründen und um im untersten Bereich das Einführen eines Schiebemittels in Richtung S zu ermöglichen, mit drei Wandstegen 10 ausgebildet. Auf der der Seitenwand 9 gegenüberliegenden Seite 8 ist der obere Bereich dieser Seitenwand noch erkennbar, der weitgehend fluchtend in den Randbereich der anderen Seitenwände übergeht. Diese Seitenwand 8 kann manuell oder automatisch geöffnet werden, wie dies z.B. in der Fig. 5 dargestellt ist.

Der Transportbehälter 1 weist im Beispiel im Deckenbereich einen Deckel 6 auf, der mit kreisförmigen Pipettieröffnungen 16 ausgestattet ist. Diese Pipettieröffnungen 16 sind den darunter zu liegen kommenden Verschlüssen, Stopfen oder Folienabschlüssen der entsprechenden Probenflaschen 2 zugeordnet.

Da der obere Randbereich des Transportbehälters 1 geringfügig über die Fläche des Deckels 6 hinausragt, ist es in diesem Fall möglich, z.B. nach dem Hochschieben der Seitenwand 8 diese weitgehend formschlüssig in der oberen Ablagefläche 19 auf dem Deckel 6 platzieren zu können.

Der Deckel 6 selbst ist über eine rückwärtige Scharnierachse an den Scharnieren 13, gegebenenfalls zusammen mit der Seitenwand 8, aufschwenkbar.

In der Verschlussstellung des Transportbehälters 1, wie sie in Fig. 1 gezeigt ist, rastet der Deckel form- und kraftschlüssig an einer komplementären Arretierung 17 mit dem oberen Rand der Seitenwand 15 ein.

Das Scharnier 13 mit Bolzen ist so gestaltet, dass eine Abflachung vorhanden ist, die z.B. nach einer Drehung des Deckels um 180 Grad, ein Lösen des Deckels vom Transportbehälter 1 ermöglicht.

Im Hinblick auf ein automatisches Entleeren wird bei diesem Transportbehälter nach Öffnung der Seitenwand 8 ein Schiebemittel in die Schiebeöffnung 11 eingebracht, wobei die Probenflaschen zusammen in Richtung des Pfeiles S auf die geöffnete Seite ausgeschoben werden können.

Im Bereich der Seitenwände 5 ist z.B. eine Vertiefung 18 vorgesehen, in die eine Codier-Kennung oder ein TAG zur Identifizierung des Transportbehälters 1 angebracht werden kann.

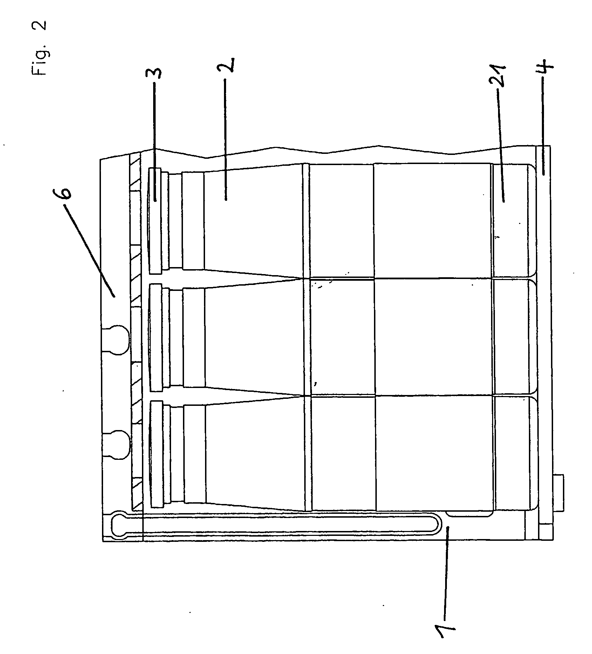

Die Figur 2 zeigt eine bruchstückartige schematische Darstellung eines Transportbehälters 1 mit Deckel 6. Die Probenflaschen 2 ruhen auf der inneren ebenen Abstellfläche des Bodens 4. Geeigneterweise weist jede Probenflasche 2 einen unteren Sockel 21 als magnetisierbaren Standsockel oder zumindest einen magnetisch leitenden Magnetsockel auf. Dieser magnetisierbare Sockel 21 ist im Hinblick auf das Einbringen und Ausschieben aus dem Transportbehälter 1 unbeachtlich, jedoch für die nachfolgende automatische Weiterleitung von großem Vorteil.

Die Probenflaschen 2 weisen kopfseitig einen Verschluss als Stopfen 3 oder Folie auf, um die bestmögliche Reinheit für die übernommende oder zu übernehmende Probenflüssigkeit sicherzustellen.

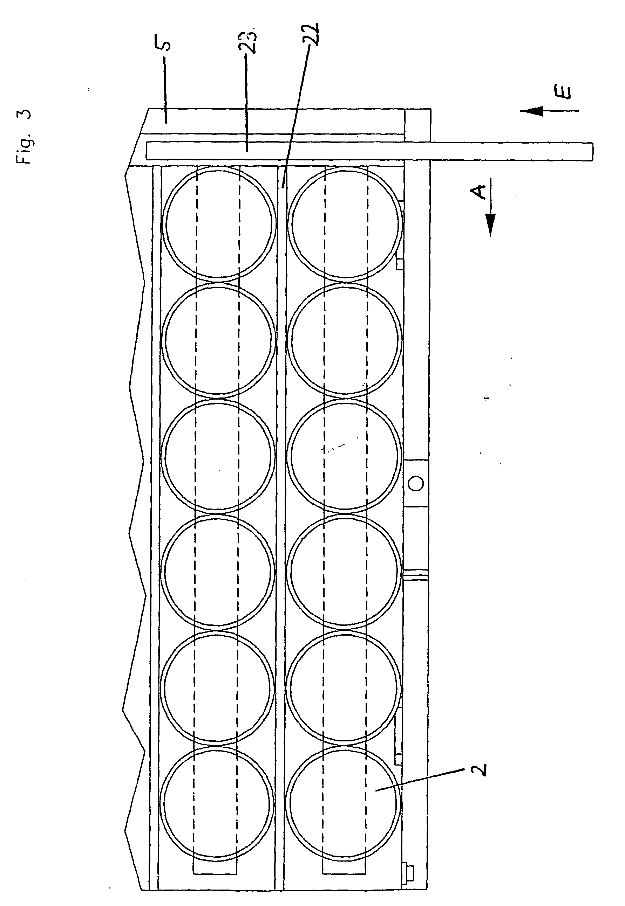

Figur 3 zeigt bruchstückartig und schematisch einen Transportbehälter 1 mit entferntem Deckel.

Wesentlich ist hierbei, dass die Längsreihen der Probenflaschen 2 in Ausschubrichtung A gegenseitig durch einen kleinen Bodensteg 22 geführt sind. Dies verbessert die Platzierungsmöglichkeit der Probenflaschen 2 und erleichtert ein reihenweises Ausschieben von Probenflaschen. Die Schiebeinrichtung kann, wie in der Fig. 3 angedeutet, eine flache Platte 23 oder ein Stab sein, der zunächst in Richtung E eingeführt wird und abhängig von der eingeführten Tiefe, im gezeigten Beispiel nur die zwei dargestellten Reihen der Probenflaschen 2, bei geöffneter Seitenwand 8 ausschieben kann.

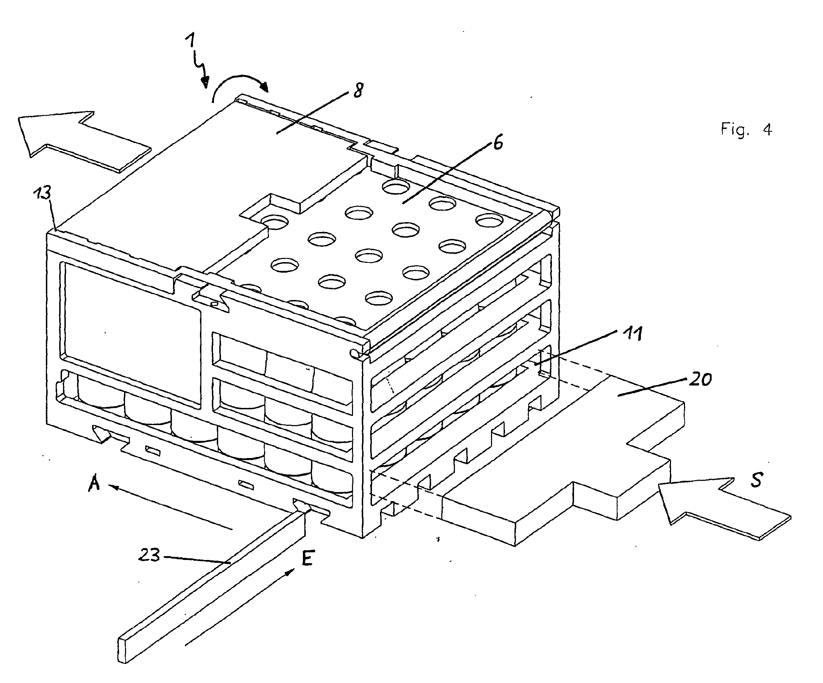

In Figur 4 ist schematisch das Prinzip des Ausschiebens aus einem Transportbehälter 1 dargestellt. Die Seitenwand 8 ist z.B. um das Scharnier 13 nach oben geschwenkt und parallel zum Deckel 6 platziert.

Im unteren Bereich des Transportbehälters 1 ist eine Schiebeöffnung 11 vorgesehen, in die ein Schieber 20 in Richtung des Pfeiles S eingeführt wird. Der Schieber 20 gelangt im Transportbehälter 1 mit den dort vorhandenen Probenflaschen in Schiebekontakt und kann auf diese Weise durch die Öffnung der nach oben gekippten Seitenwand 8 die Probenflaschen ausschieben.

Alternativ kann der Schieber 20 auch durch eine flache Platte 23 realisiert werden, die zunächst in Pfeilrichtung E zwischen Transportbehälter 1 und Probenflaschen eingeführt wird (siehe Fig. 3). Anschließend erfolgt das Verschieben der Platte 23 in Pfeilrichtung A, wodurch die Probenflaschen ausgeschoben werden.

In der Darstellung nach Fig. 5 ist ein geringfügig modifizierter Transportbehälter 30 gezeigt, der eine größere Anzahl Probenflaschen 2 aufnehmen kann. Die Seitenwand 8 ist in der Darstellung geöffnet, wozu Führungsnuten 32 im seitlichen Bereich komplementär zu entsprechenden Führungszapfen im Randbereich der Seitenwand ein geführtes Öffnen ermöglichen.

Zum Öffnen wird die Seitenwand 8 zuerst senkrecht nach oben geschoben und anschlißend auf den Deckel zurückgeklappt. Durch diese Öffnungsweise ist es möglich den Transportbehälter nahe an eine Fördereinrichtung heranzuführen ohne dass der Schwenkbereich für das Öffnen frei bleiben muss. Würde die Seitenwand 8 zuerst geklappt werden, würde das Öffnen der Wand 8 von davor stehenden Flaschen behindert werden. Beim Klappen der Seitenwand 8 nach unten würde zusätzlich eine weitere Ebene geschaffen, über die die Flaschen dann zu transportieren wären.

Gleiche Bezugszeichen entsprechen hierbei konstruktiven Gegebenheiten, wie sie vorher erwähnt worden sind.
Zur Bildung von größeren Transportbehälter-Einheiten weist der Transportbehälter 30 im Boden- und Deckenbereich trapezartig ausgebildete Verbindungselemente 34 auf. Mittels dieser Verbindungselemente 34 ist es möglich, einen horizontal neben dem Transportbehälter 30 angeordneten zweiten Transportbehälter 30 zu einer stabilen, größeren Transport-einheit fest zu verbinden.
In gleicher Weise kann auch die Stapelbarkeit der Transportbehälter 1, 30 realisiert werden. Hierzu ist zweckmäßigerweise im Bereich der vertikalen Ecken eine Arretiervorrichtung über z.B. halbkreisförmige Vertiefungen mit entsprechenden komplementären kugelförmigen Füßen möglich.

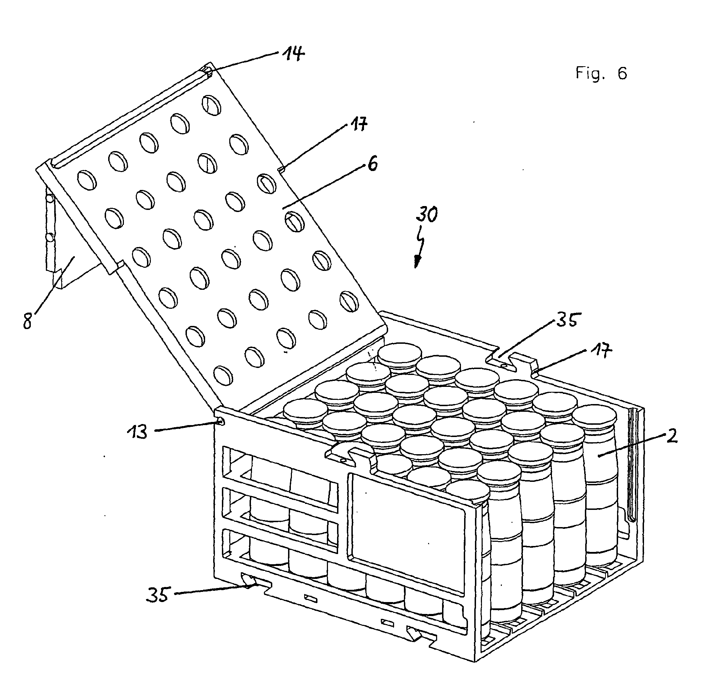

Die Darstellung nach Figur 6 zeigt einen geöffneten Transportbehälter 30. Der Deckel 6 ist hierbei über das Scharnier 13 zurückgeschlagen und weist in diesem Fall als Einheit die Seitenwand 8 auf, die angelenkt am oberen Ende z.B. über eine Zapfenverbindung bzw. ein Seitenwandscharnier 14, vorgesehen ist.

Im unteren Bodenbereich und im oberen Randbereich sind etwa trapezförmige Aufnahmen 35 dargestellt, in die entsprechene Verbindungselemente 34 (Fig. 5) zur Kaskadierung und Verbindung mit weiteren Transportbehältern einsetzbar sind.

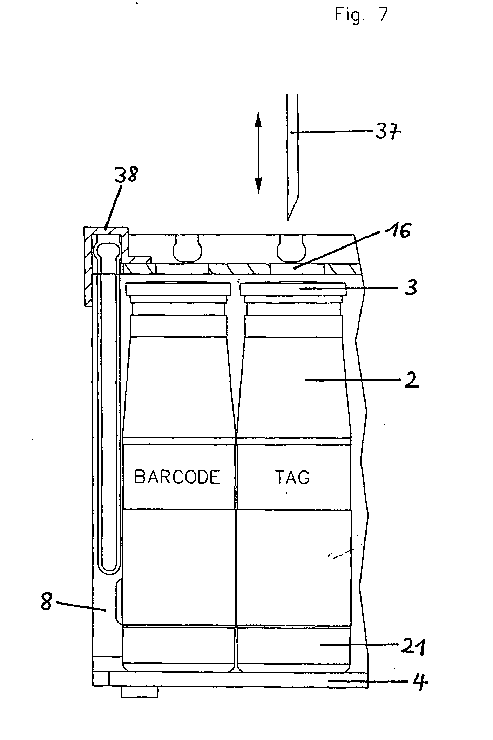

Die Figur 7 zeigt schematisch einen Vertikalschnitt durch einen Transportbehälter in bruchstückartiger Darstellung. Es sind zwei mit einem Barcode bzw. einem TAG versehene Probenflaschen 2 dargestellt, die einen oberen Stopfen 3 als Verschluss aufweisen. Die Probenflaschen 2 haben im unteren Bereich einen metallischen Sockel 21.

Die Seitenwand 8, welche geöffnet werden kann, ist auf dieser Seite zum Boden arretiert dargestellt, wobei im oberen Bereich des Seitenwandscharnieres 14 ein Siegel 38, als Klebesiegel oder Kunststoffsiegel gegenüber dem Deckel 6 und der Seitenwand 8 aufgebracht ist. Eine Beschädigung des Siegels 38 lässt daher für die genommenen Proben eine mögliche Manipulierung erkennen.

Die z.B. aus einem Elastomer bestehenden Stopfen 3 der Probenflaschen 2 sind entsprechenden Pipettieröffnungen 16 im Deckel 6 zugeordnet. Auf diese Weise ist es möglich, die Proben z.B. durch Herabfahren einer Injektionsnadel 37 nach dem Durchstechen des Stopfens 3 in die Probenflasche 2 automatisch abzugeben. Nach genommener Probe wird die Injektionsnadel nach oben zurückgefahren. Auch eine Probeentnahme aus der jeweiligen Probenflasche 2 wäre in dieser Weise denkbar.

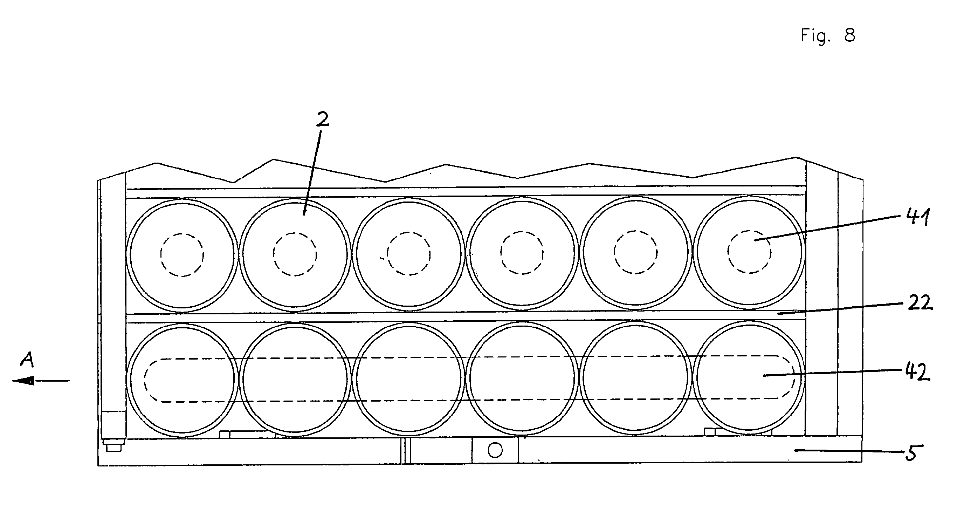

Die Darstellung nach Figur 8 entspricht weitgehend dem in Figur 3 gezeigten bruchstückartigen Teil eines Transportbehälters 1. Im Bodenbereich des Transportbehälters sind bei der oberen Reihe kreisförmige Öffnungen 41 mit unterbrochenen Strichen dargestellt. Diese Öffnungen 41 ermöglichen ein Anheben oder Absenken der entsprechenen Probenflaschen 2 mittels von der Bodenseite her eingeführter, z.B. magnetischer, Stößel.
Die in der untersten Reihe gezeigte Langlochöffnung 42 ermöglicht zudem ein Verschieben der Probenflaschen 2 z.B. in Richtung A. Der Durchmesser der Öffnung 41 bzw. die Breite des Langloches 42 sind so bemessen, dass sie kleiner sind als der Durchmesser einer Probenflasche 2, damit eine ausreichende Standfläche für die Probenflasche gewährleistet ist.

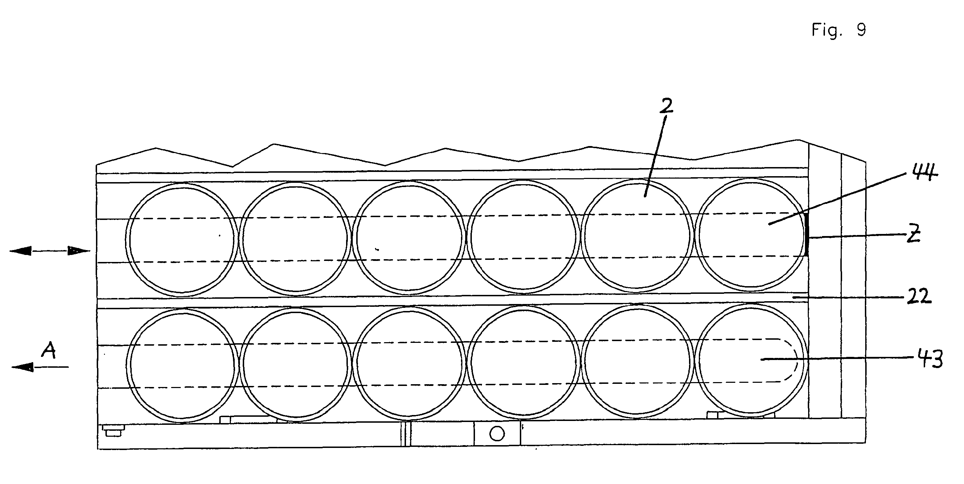

Die Darstellung nach Figur 9 zeigt weitere Alternativen zur bodenseitigen Öffnung. So kann, wie in der untersten Reihe gezeigt, eine U-förmige Öffnung 43 im Boden 4 vorgesehen sein, die es erlaubt, z.B. mittels eines magnetischen Schiebers, die Probenbehälter aus dem Transportbehälter heraus- bzw. hineinzubeförderen.

Die rechteckige Langlochöffnung 44 erlaubt diese Maßnahme entsprechend der Pfeildarstellung mit einem einfachen mechanischen Schieber, der z.B. in der Position Z angreift und die Flaschen in Richtung A ausschiebt.

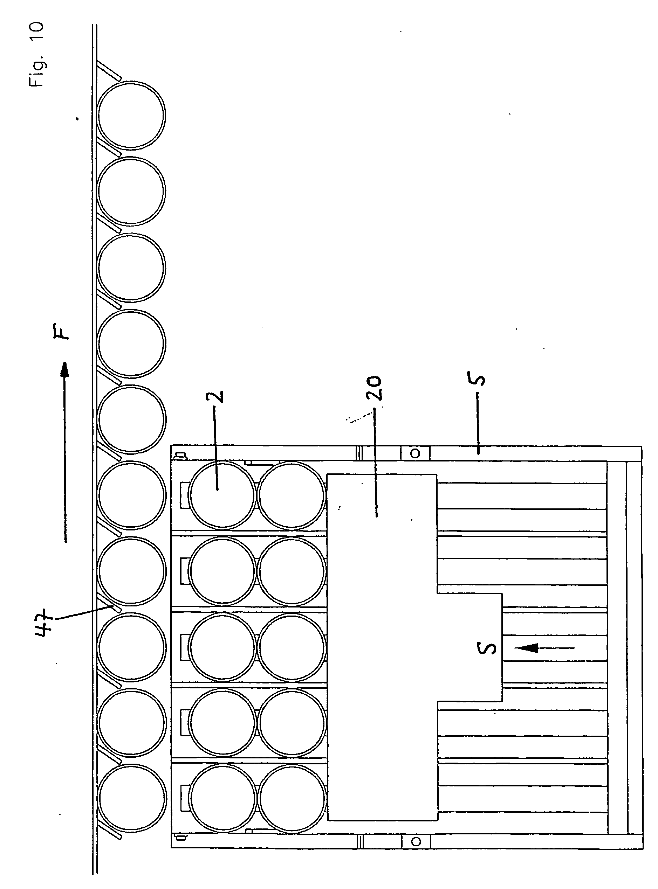

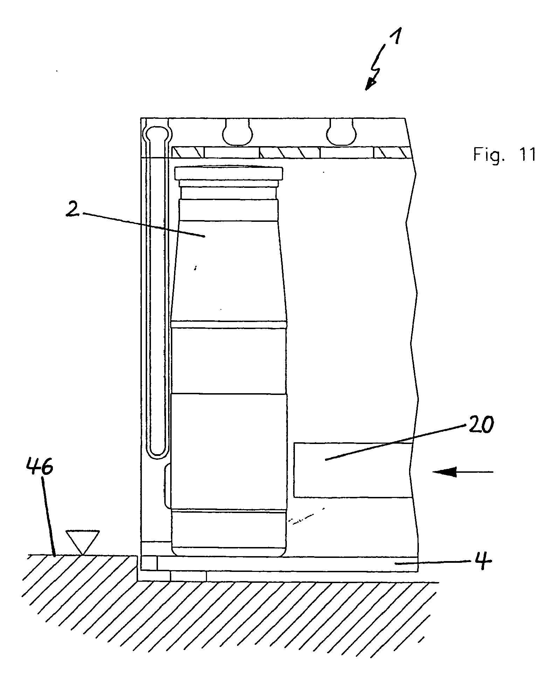

Die schematische Darstellung nach Figur 10 zeigt das Ausschieben von Probenflaschen 2 aus einem Transportbehälter, wobei der Schieber 20 in Richtung des Pfeiles S kraftschlüssig auf die Probenflaschen einwirkt. Die Probenflaschen 2 werden hierbei auf weitgehend gleichem Niveau (Fig. 11) auf z.B. einen Schneckenförderer 47 getaktet übergeben und in Förderrichtung F weitergeleitet.

In Fig. 11 ist die zweckmäßige Übergabesituation und das Förderniveau 46 dargestellt, auf dem Probenflaschen 2 aus einem Transportbehälter 1 mittels des Schiebers 20 ausgeschoben werden. Das Bodenniveau 4 und das Förderniveau 46 liegen hierbei in gleicher Ebene.

## Patentansprüche

1. Transportbehälter für Probenflaschen oder flaschenartige Behälter,
mit Bodenbereich (4), Seitenbereichen (5, 9, 8) und gegebenenfalls Deckenbereich (6),
mit einer im Wesentlichen rechteckförmigen, ebenen Abstellfläche für die Probenflaschen (2) im Bodenbereich (4),
mit mindestens seitlichen, die Abstellfläche begrenzenden Seitenwänden (5, 8, 9) im Seitenbereich,
wobei mindestens eine Seitenwand (8), von einer Verschlussposition in eine den Bereich der Seitenwand freigebende Öffnungsposition überführbar ist, wobei
die zu öffnende Seitenwand (8) mindestens im Bereich der freizugebenden Öffnung verschiebbar ausgelegt ist, und
**dadurch gekennzeichnet,**
**dass** Bodenbereich (4), Seitenbereich (5, 9, 8) und/oder Deckenbereich (6) so ausgebildet sind,
**dass** die Probenflaschen (2) einzeln, als Reihe oder als Block aus dem Transportbehälter (1; 30) zur Weiterförderung ausschiebbar sind.

2. Transportbehälter nach Anspruch 1,
**dadurch gekennzeichnet,**
**dass** die zu öffnende Seitenwand (8), insbesondere zur Seite oder nach oben, verschiebbar und klappbar oder verschwenkbar ausgelegt ist.

3. Transportbehälter nach Anspruch 1 oder 2,
**dadurch gekennzeichnet,**
**dass** ein fixier- und entfernbarer Deckel (6), insbesondere mit Pipettieröffnungen (16), die den Verschlüssen (3) der Probenflaschen (2) zugeordnet sind, vorgesehen ist.

4. Transportbehälter nach einem der Ansprüche 1 bis 3,
**dadurch gekennzeichnet,**
**dass** der die Abstellfläche für die Probenflaschen (2) bildende Boden (4) des Transportbehälters (1; 30) den Probenflaschen (2) zugeordnete Bodenöffnungen (41, 42, 43, 44) aufweist, die mindestens quer zur Ausschieberichtung (A) eine kleinere Abmessung als der bodenseitige Durchmesser oder die entsprechende Abmessung der Probenflaschen (2) aufweist.

5. Transportbehälter nach Anspruch 4,
**dadurch gekennzeichnet,**
**dass** die Bodenöffnungen kreisförmig (41) U-förmig (43, 44) in Ausschieberichtung (A) ausgebildet sind.

6. Transportbehälter nach einem der Ansprüche 1 bis 5,
**dadurch gekennzeichnet,**
**dass** bodenseitige und/oder deckenseitige Führungsstege (22) für die Probenflaschen (2) in Ausschieberichtung (A) vorhanden sind.

7. Transportbehälter nach einem der Ansprüche 1 bis 6,
**dadurch gekennzeichnet,**
**dass** ein oder mehrere Seitenwände (5, 9) über ihre Breite vorgesehene Wandstege (10) mit einem freien Abstand dazwischen aufweisen.

8. Transportbehälter nach einem der Ansprüche 1 bis 7,
**dadurch gekennzeichnet,**
**dass** die zu öffnende Seitenwand (8) und der Deckel (6) eine verbundene, lösbare Einrichtung bilden, die insbesondere relativ zueinander und/oder gemeinsam verschiebbar, verschwenkbar und/oder verkippbar angeordnet ist und mit dem Transportbehälter (1; 30) fixiert oder lösbar verbunden ist.

9. Transportbehälter nach einem der Ansprüche 1 bis 8,
**dadurch gekennzeichnet,**
**dass** mehrere, insbesondere über Eck stehende Seitenwände (5, 8), öffenbar ausgebildet und angeordnet sind.

10. Transportbehälter nach einem der Ansprüche 1 bis 9,
**dadurch gekennzeichnet,**
**dass** die öffenbare Seitenwand (8) nach oben verschieb-oder verschwenkbar ist und weitgehend formschlüssig am/im Deckel (6) platzierbar ist.

11. Transportbehälter nach einem der Ansprüche 1 bis 10,
**dadurch gekennzeichnet,**
**dass** dieser stapelbar und/oder kaskadierbar ausgebildet ist.

12. Transportbehälter nach einem der Ansprüche 1 bis 11,
**dadurch gekennzeichnet,**
**dass** dieser mindestens etwas höher als die Probenflasche (2) gebildet ist.

13. Transportbehälter nach einem der Ansprüche 1 bis 12,
**dadurch gekennzeichnet,**
**dass** mehrere Transportbehälter (1; 30) horizontal und/oder vertikal form- und/oder kraftschlüssig, insbesondere selbstrastend, zu größeren Transportbehälter-Einheiten zusammenstellbar sind.

14. Transportbehälter nach einem der Ansprüche 1 bis 13,
**dadurch gekennzeichnet,**
**dass** einsetzbare Gitter mit weitgehend formschlüssiger Führung der Probenflaschen (2) vorgesehen sind.

15. Transportbehälter nach einem der Ansprüche 1 bis 14,
**dadurch gekennzeichnet,**
**dass** der Deckel (6) sowie die Seitenwand (8) mit dem Transportbehälter (1; 30) versiegelbar (38) sind.

16. Transportbehälter nach einem der Ansprüche 1 bis 15,
**dadurch gekennzeichnet,**
**dass** die Probenflaschen (2) bodenseitig magnetisch leitfähig mit Kappe (21) oder Scheibe ausgebildet sind und eine Identifizierungskennung aufweisen.

17. Transportbehälter nach einem der Ansprüche 1 bis 16,
**dadurch gekennzeichnet,**
**dass** dieser eine Markierung oder einen TAG aufweist.

18. Verfahren zum definierten Entleeren eines mindestens vier Seitenwände (5, 8, 9) aufweisenden Transportbehälters (1; 30) mit auf dessen Bodenfläche (4) vorgesehenen Behältern, speziell Probenflaschen, insbesondere nach einem der Ansprüche 1 bis 17,
**dadurch gekennzeichnet,**
**dass** mindestens eine Seitenwand (8), insbesondere nach außen oder oben, verschoben wird, und
**dass** die Behälter (2) einzeln, reihenweise oder blockweise bei geöffnetem Bereich der Seitenwand (8) durch diesen Bereich schwerkraftmäßig und/oder kraftbeaufschlagt ausgeschoben (S) werden.

19. Verfahren nach Anspruch 18,
**dadurch gekennzeichnet,**
**dass** das Ausschieben (5) mittels einer, insbesondere im unteren Bereich der Behälter (2) angreifenden Schiebeeinrichtung (20, 23), durchgeführt wird.

20. Verfahren nach Anspruch 18 oder 19,
**dadurch gekennzeichnet,**
**dass** zum schwerkraftmäßigen Ausschieben der Transportbehälter (1; 30) in eine schiefe Ebene gebracht wird.

21. Verfahren nach einem der Ansprüche 18 bis 20,
**dadurch gekennzeichnet,**
**dass** das Ausschieben (5) der Behälter oder Probenflaschen (2) mittels der im Wesentlichen in einer parallelen Ebene zur geöffneten Seitenwand (8) vorgesehenen Schiebeeinrichtung (20) von der gegenüberliegenden Seitenwand (9) mittels einer in Schieberichtung (S) wirkenden oder seitlich dazu eingeführten Schiebefläche (23), oder durch den Boden (4) des Transportbehälters (1; 30) eingebrachte Schiebemittel, durchgeführt wird.

## Claims

1. Transport container for sample bottles or bottle-like receptacles,
with a bottom area (4), side areas (5, 9, 8) and optionally a cover area (6),
with a substantially rectangular, planar standing surface for the sample bottles (2) in the bottom area (4),
with at least lateral side walls (5, 8, 9) in the side area bounding the standing surface, at least one side wall (8) being transferable from a closure position into an opening position freeing the area of the side wall (8) to be opened, at least in the area of the opening to be freed, having a displaceable construction,
**characterized in that**
the bottom area (4), side area (5, 9, 8) and/or cover area (6) are constructed in such a way that the sample bottles (2) can be slid out of the transport container (1; 30) individually, as a row or as a block for further conveying purposes.

2. Transport container according to claim 1,
**characterized in that**
the side wall (8) to be opened, is designed so as to be displaceable and foldable or pivotable more particularly sideways or upwards.

3. Transport container according to claim 1 or 2,
**characterized in that**
there is a fixable and removable cover (6), particularly with pipetting openings (16), which are associated with the closures (3) of the sample bottles (2).

4. Transport container according to one of the claims 1 to 3,
**characterized in that**
the bottom (4) of the transport container (1; 30) forming the standing surface for the sample bottles (2) have bottom openings (41, 42, 43, 44) associated with the latter and which at least transversely to the sliding out direction (A) have a smaller size than the bottom-side diameter or the corresponding size of the sample bottles (2).

5. Transport container according to claim 4,
**characterized in that**
the bottom openings are constructed in circular (41) U-shaped manner (43, 44) in the sliding out direction (A).

6. Transport container according to one of the claims 1 to 5,
**characterized in that**
there are bottom-side and/or cover-side guide webs (22) for the sample bottles (2) in sliding out direction (A).

7. Transport container according to one of the claims 1 to 6,
**characterized in that**
one or more side walls (5, 9) have wall webs (10) provided over the width thereof and with a free spacing between them.

8. Transport container according to one of the claims 1 to 7,
**characterized in that**
the side wall (8) to be opened and the cover (6) form a linked, releasable device, which is in particular displaceable, pivotable or tilting relative to one another and/or jointly and is fixed or detachably connected to the transport container (1; 30).

9. Transport container according to one of the claims 1 to 8,
**characterized in that**
there are several openable and in particular crosswise-positioned side walls (5, 8).

10. Transport container according to one of the claims 1 to 9,
**characterized in that**
he openable side wall (8) is displaceable or pivotable upwards and is placed in a substantially positive manner on or in the cover (6).

11. Transport container according to one of the claims 1 to 10,
**characterized in that**
it is constructed in stackable and/or cascadable manner.

12. Transport container according to one of the claims 1 to 11,
**characterized in that**
it is at least somewhat higher than the sample bottles (2).

13. Transport container according to one of the claims 1 to 12,
**characterized in that**
several transport containers (1; 30) can be assembled in horizontal and/or vertical, positive and/or non-positive and in particular self-locking manner to form larger transport container units.

14. Transport container according to one of the claims 1 to 13,
**characterized in that**
there are insertable grids with a substantially positive guidance of the sample bottles (2).

15. Transport container according to one of the claims 1 to 14,
**characterized in that**
the cover (6) and side wall (8) can be sealed (38) to the transport container (1; 30).

16. Transport container according to one of the claims 1 to 15,
**characterized in that**
at the bottom, the sample bottles (2) are constructed in magnetically conductive manner with cap (21) or disk and have an identification.

17. Transport container according to one of the claims 1 to 16,
**characterized in that**
it has a marking or tag.

18. Method for a clearly defined emptying of a transport container (1; 30) having at least four side walls (5, 8, 9) with receptacles, specifically sample bottles provided on its bottom plane (4), particularly according to one of the claims 1 to 17,
**characterized in that**
at least one side wall (8) can be displaced more particularly outwards or upwards and
that the receptacles (2) individually, rowwise or block-wise can be slid out through an area of the side wall (8) opened under gravity and/or force action (S).

19. Method according to claim 18,
**characterized in that**
the sliding out (5) takes place by means of a sliding device (20, 23) more particularly acting in the lower area of the receptacles (2).

20. Method according to claim 18 or 19,
**characterized in that**
for the gravity-based sliding out the transport container (1; 30) is brought into an oblique plane.

21. Method according to one of the claims 18 to 20,
**characterized in that**
the sliding out (5) of the receptacles or sample bottles (2) is carried out by means of the sliding device (20) essentially provided in a parallel plane to the opened side wall (8) from the facing side wall (9) by means of a sliding surface (23) acting in sliding direction (S) or inserted laterally thereto, or sliding means introduced through the bottom (4) of the transport container (1; 30).

## Revendications

1. Caisson de transport pour bouteilles à échantillon ou récipients de type bouteille, comprenant :
une zone de fond (4), des zones latérales (5, 9, 8) et le cas échéant une zone de couvercle (6),
une surface de rangement plane pour l'essentiel rectangulaire pour les bouteilles à échantillon (2) dans la zone de fond (4),
au moins des parois latérales (5, 8, 9) délimitant la surface de rangement dans la zone latérale, au moins une paroi latérale (8) pouvant être transférée d'une position de fermeture dans une position d'ouverture dégageant la zone de la paroi latérale, la paroi latérale (8) à ouvrir étant conçue coulissante au moins dans la zone de l'ouverture à dégager,
**caractérisé en ce que**
la zone de fond (4), la zone latérale (5, 9, 8) et/ou la zone de couvercle (6) sont configurées pour permettre de pousser hors du caisson de transport (1; 30) les bouteilles à échantillon (2) individuellement, par rangées ou en bloc pour continuer le transport.

2. Caisson de transport selon la revendication 1,
**caractérisé en ce que**
la paroi latérale (8) à ouvrir est conçue coulissante et rabattable ou pivotante, en particulier vers le côté ou vers le haut.

3. Caisson de transport selon la revendication 1 ou 2,
**caractérisé en ce qu'**
un couvercle (6) pouvant être fixé et enlevé est en particulier muni d'orifices à pipeter (16) associés aux fermetures (3) des bouteilles à échantillon.

4. Caisson de transport selon l'une quelconque des revendications 1 à 3,
**caractérisé en ce que**
le fond (4) du caisson de transport (1; 30) formant la surface de rangement pour les bouteilles à échantillon (2) présente des ouvertures de fond (41, 42, 43, 44) associées aux bouteilles à échantillon (2) et présentant au moins transversalement à la direction de sortie (A) une plus petite dimension que le diamètre côté fond ou que la dimension des bouteilles à échantillon (2).

5. Caisson de transport selon la revendication 4,
**caractérisé en ce que**
les ouvertures de fond présentent la forme de cercle (41) et de U (43, 44) dans la direction d'extraction (A).

6. Caisson de transport selon l'une quelconque des revendications 1 à 5,
**caractérisé en ce que**
des nervures de guidage (22) sont prévues côté fond et/ou côté couvercle pour les bouteilles à échantillon (2) dans la direction d'extraction (A).

7. Caisson de transport selon l'une quelconque des revendications 1 à 6,
**caractérisé en ce qu'**
une ou plusieurs parois latérales (5, 9) présentent des nervures de paroi (10) prévues sur leur largeur avec un espace libre entre elles.

8. Caisson de transport selon l'une quelconque des revendications 1 à 7,
**caractérisé en ce que**
la paroi latérale (8) à ouvrir et le couvercle (6) forment un dispositif relié amovible, en particulier coulissant, pivotant et/ou basculant l'un par rapport à l'autre et/ou conjointement, et relié au caisson de transport (1; 30) de façon fixe ou amovible.

9. Caisson de transport selon l'une quelconque des revendications 1 à 8,
**caractérisé en ce que**
plusieurs parois latérales (5, 8) disposées en particulier rectangulaire peuvent être ouvertes.

10. Caisson de transport selon l'une quelconque des revendications 1 à 9,
**caractérisé en ce que**
la paroi latérale (8) à ouvrir peut être coulissée ou pivotée vers le haut et placée sensiblement par complémentarité de formes sur/dans le couvercle (6).

11. Caisson de transport selon l'une quelconque des revendications 1 à 10,
**caractérisé en ce qu'**
il est formé de façon à pouvoir être empilé ou positionné en cascade.

12. Caisson de transport selon l'une quelconque des revendications 1 à 11,
**caractérisé en ce qu'**
il est au moins légèrement plus haut que la bouteille à échantillon (2).

13. Caisson de transport selon l'une quelconque des revendications 1 à 12,
**caractérisé en ce qu'**
on peut rassembler plusieurs caissons de transport (1; 30) horizontalement et/ou verticalement par complémentarité de formes et/ou de forces, en particulier de façon autobloquante, en unités de caissons de transport plus grandes.

14. Caisson de transport selon l'une quelconque des revendications 1 à 13,
**caractérisé en ce que**
des grilles insérables sont prévues avec guidage sensiblement par complémentarité de formes des bouteilles à échantillon (2).

15. Caisson de transport selon l'une quelconque des revendications 1 à 14,
**caractérisé en ce que**
le couvercle (6) ainsi que la paroi latérale (8) peuvent être scellés (38) avec le caisson de transport (1; 30).

16. Caisson de transport selon l'une quelconque des revendications 1 à 15,
**caractérisé en ce que**
côté fond les bouteilles à échantillon (2) sont munies d'un capuchon (21) ou d'une plaque de façon magnétiquement conductrice et présentent une marque d'identification.

17. Caisson de transport selon l'une quelconque des revendications 1 à 16,
**caractérisé en ce qu'**
il présente un marquage ou un code à barres (TAG).

18. Procédé de déchargement défini d'un caisson de transport (1; 30) présentant au moins quatre parois latérales (5, 8, 9) avec des récipients, en particulier des bouteilles à échantillon, prévus sur la surface de fond (4) de celui-ci, en particulier selon l'une quelconque des revendications 1 à 17,
**caractérisé en ce qu'**
au moins une paroi latérale (8) est déplacée en particulier vers l'extérieur ou vers le haut, et
lorsque la zone de la paroi latérale (8) est ouverte, les récipients (2) sont poussés (S) hors de cette zone par force de gravité et/ou sollicités par force individuellement, par rangées ou en bloc.

19. Procédé selon la revendication 18,
**caractérisé en ce que**
l'extraction (5) est effectuée au moyen d'un dispositif de poussée (20, 23) intervenant en particulier dans la zone inférieure des récipients (2).

20. Procédé selon la revendication 18 ou 19,
**caractérisé en ce que**
pour l'extraction par force de gravité, le caisson de transport (1 ; 30) est positionné dans un plan incliné.

21. Procédé selon l'une quelconque des revendications 18 à 20,
**caractérisé en ce que**
l'extraction (5) des récipients ou bouteilles à échantillon (2) s'effectue au moyen du dispositif de poussée (20) prévu pour l'essentiel dans un plan parallèle à la paroi latérale (8) ouverte, depuis la paroi latérale (9) opposée au moyen d'une surface de poussée (23) agissant dans la direction de poussée (S) ou introduite latéralement à celle-ci, ou par des moyens de poussée introduits à travers le fond (4) du caisson de transport (1; 30).
